# EUROPÄISCHE PATENTSCHRIFT

(11) **EP 1 343 439 B1**
(45) Veröffentlichungstag und Bekanntmachung des Hinweises auf die Patenterteilung: **01.09.2004**
(21) Anmeldenummer: 01989051.6
(22) Anmeldetag: 18.12.2001
(51) Int. Cl.: A61F 2/38

(54) **KNIEGELENKENDOPROTHESE**
KNEE JOINT ENDOPROSTHESIS
ENDOPROTHESE DE L'ARTICULATION DU GENOU

(30) Priorität: 20.12.2000 DE 10065940
(43) Veröffentlichungstag der Anmeldung: 17.09.2003
(73) Patentinhaber: ESKA Implants GmbH & Co., 23556 Lübeck (DE)
(72) Erfinder: GRUNDEI, Hans, 23556 Lübeck (DE); THOMAS, Wolfram, I-00135 Rom (IT)
(74) Vertreter: Fuchs Mehler Weiss & Fritzsche
(86) Internationale Anmeldenummer: PCT/EP2001/014968
(87) Internationale Veröffentlichungsnummer: WO 2002/054993

(56) Entgegenhaltungen:
- EP-A- 0 864 306

## Beschreibung

Die vorliegende Erfindung betrifft eine Kniegelenkendoprothese gemäβ dem Oberbegriff von Anspruch 1. Eine solche kniegelenkendoprothese ist aus dem Dokument EP-A-0864-306 bekannt.

Derartige Kniegelenkendoprothesen sind in vielfältiger Ausführung bekannt und bereits im klinischen Einsatz. Grundsätzlich ist man bemüht gewesen, den natürlichen, anatomischen Bewegungsablauf in einem solchen künstlichen Gelenk nachzuempfinden, um beispielsweise den Bandapparat, sofern er noch intakt ist, nicht über Gebühr zu überlasten.

Bekannte anatomische Kniegelenkendoprothesen bestehen üblicherweise aus einem mit einem im Femurknochen zu verankernden Stielteil verbindbaren Femurteil, das mit zwei Gleitkufen versehen ist, die ventral über eine Brücke miteinander verbunden sind.

Es gibt auch Ausführungsformen, bei denen die Gleitkufen einstückig mit dem Stielteil ausgebildet sind. Es kommt aber im Rahmen der vorliegenden Erfindung nicht darauf an, ob das Femurteil nun ein- oder mehrteilig ausgebildet ist.

Darüber hinaus besteht eine anatomische Kniegelenkendoprothese üblicherweise aus einem mit einem im Tibiaknochen zu verankernden Stielteil zusammenwirkenden Tibiaplateauteil, vorzugsweise aus einem Kunststoff wie hochverdichteten Polyehtylen, in welchen zwei Gleitbahnen ausgebildet sind, auf denen die Gleitkufen des Femurteils eine Abroll- und Gleitbewegung ausführen können.

Entsprechend der physiologischen Vorgabe nimmt bei einem solchen anatomischen Kniegelenk - ausgehend von der Strecklage des Gelenks - mit zunehmender Beugelage die Möglichkeit zu, daß das Femurteil um das Tibiateil herum rotiert. Gleichzeitig vollführt das Femurteil auf dem Tibiaplateauteil eine schubladenartige Bewegung nach dorsal.

Andere Konzepte beschreiten nicht den rein anatomischen Weg, sondern versuchen mittels eines rein technischen Weges, den Patientenerfordernissen gerecht zu werden. So ist beispielsweise in der nicht vorveröffentlichten DE-100 60 850 der Anmelderin ein Set zur Erstellung eines Tibiateils einer eingangs erwähnten Kniegelenkendoprothese vorgeschlagen worden, die einen Sockelteil sowie wenigstens zwei lose auf das Sockelteil setzbare Kunststoffauflagen umfaßt, in denen die Gleitbahnen für die Gleitkufen eines Femurteils der Endoprothese ausgeformt sind. Die Gleitbahnen der zweiten Kunststoffauflage sind gegenüber jenen der ersten Kunststoffauflage um eine Strecke von ventral nach dorsal versetzt, die zwischen 0 und 12 mm liegen kann. Mit diesem Set wird insbesondere dem Zustand des Bandapparates des Patienten Rechnung getragen und der Operateur wird aus dem Set ein entsprechendes patientenindividuelles Implantat zusammenstellen. Dieser Ansatz ist - wie ausgeführt - rein technisch und bietet eine diskutable Alternative zu den anatomischen Kniegelenkendoprothesen.

Letztendlich ist die wissenschaftliche Diskussion über Vor- und Nachteile der einen oder anderen Lösung noch nicht abgeschlossen. Dem einen wie dem anderen Konzept haften je nach Indikation Vor- und Nachteile für den jeweils zu versorgenden Patienten an.

Vor diesem Hintergrund ist es nun die Aufgabe der vorliegenden Erfindung, eine eingangs erwähnte Kniegelenkendoprothese so weiterzubilden, daß sowohl Aspekte der rein anatomischen Kniegelenkendoprothese wie auch der rein technischen Endoprothese realisiert werden, um so die jeweiligen Vorteile beider Arten von Endoprothesen in einer zu vereinigen bzw. zu bündeln.

Gelöst wird diese Aufgabe mit einer Kniegelenkendoprothese mit den Merkmalen des Anspruchs 1. Vorteilhafte Weiterbildungen ergeben sich aus den Unteransprüchen.

So wird grundlegend vorgeschlagen, daß das Tibiaplateauteil auf dem Stielteil mittels eines Rotationsmechanismus verschwenkbar gelagert ist und bis zu einem vorgegebenen Beugungswinkel des Kniegelenks ein Kraft- und Formenschluß zwischen Femurteil und Tibiaplateauteil wirkt, derart, daß eine Rotationsbewegung des Femurteils auf dem Stielteil im Tibiaknochen aufgrund der verschwenkbaren Lagerung des Tibiaplateauteils auf dem Stielteil ausführbar ist, und daß dem Femurteil bei zunehmender Beugung des Kniegelenks ab dem vorgegebenen Beugewinkel andererseits eine zunehmende Rotation des Femurteils gegenüber dem Tibiaplateauteil ermöglicht wird, allerdings ohne Einschaltung des Rotationsmechanismus zwischen Tibiaplateauteil und Stielteil.

Ausgehend von der Strecklage des Kniegelenks steht zunächst bei einer ersten Beugung des Knies der Bewegungsablauf eines technischen Kniegelenks im Vordergrund steht. Dabei greift vor Überschreiten eines bestimmten Beugewinkels der Kraft- und Formenschluß zwischen dem Femurteil und dem Tibiaplateauteil und die rein technisch bedingte Rotationsmöglichkeit aufgrund der Lagerung des Tibiaplateauteils auf dem Stielteil mittels eines Rotationsmechanismus gestattet die Ausführung einer Rotationsbewegung des Femurteils gegenüber dem Stielteil. Das Femurteil nimmt also bis zum Überschreiten eines gewissen Beugungswinkels das Tibiaplateauteil in seinen Bewegungen mit und vollführt mit diesem als Einheit eine Rotationsbewegung auf dem mit dem Tibiaknochen verankerten Stielteil. Es findet sodann beim Überschreiten des vorgegebenen Beugewinkels ein regelrechtes Schalten innerhalb der Endoprothese zwischen dem vorherigen technisch bedingten Bewegungsablauf und dem dann zum Tragen kommenden rein anatomischen Bewegungsablauf statt. Daher wird dieses Gelenk auch als Hybridgelenk bezeichnet, da es sowohl die anatomischen, als auch die technischen Aspekte bekannter Endoprothesen in sich vereinigt.

In einer konkreten bevorzugten Ausführungsform ist vorgesehen, daß die Gleitkufen des Femurteils zwischen sich einen von ventral nach dorsal verlaufenden Freiraum begrenzen und daß beide Gleitbahnen des Tibiaplateauteils durch zwei von dorsal nach ventral ausgerichtet verlaufende Stege getrennt sind. Dabei weist der dorsal gelegene Steg eine solche Höhe auf, daß er bis zu dem vorgegebenen Beugungswinkel des Kniegelenks in den Freiraum zwischen den Gleitkufen des Femurteils greift und so den Kraft- und Formenschluß herstellt. Der ventral gelegene Steg hingegen weist lediglich eine solche Höhe auf, daß bei zunehmender Beugung des Kniegelenks ab dem Heraustreten des dorsalen Steges aus dem Freiraum zwischen den Gleitbahnen des Femurteils, also nach dem Schalten zwischen technisch bedingter Bewegung und anatomischer Bewegung, eine zunehmende Rotation des Femurteils gegenüber dem Tibiaplateauteil ermöglicht wird, allerdings ohne Einschaltung des Rotationsmechanismus. Dies bedeutet, daß der ventral gelegene Steg eine sehr geringe Höhe hat und in erster Linie eine Führungsfunktion ausübt.

Bei dieser Ausführungsform ist die "Schaltvorrichtung" durch das Femurteil und durch das Tibiaplateauteil realisiert, und zwar in einem Fall durch den Freiraum zwischen den Gleitkufen mit entsprechenden Anlageflächen und im anderen Fall durch die Ausbildung des dorsal gelegenen Steges, dessen Seitenwände mit den Seitenkanten der Kondylen des Femurteils in Anlage kommen und den Kraftfluß übertragen.

Bevorzugt wird eine Weiterbildung, bei der der für ein Tibiaplateauteil vorgegebene Beugungswinkel im Bereich zwischen 20° und 40° liegt, bis zu dessen Überschreiten der Kraft- und Formenschluß zwischen dem Femurteil und dem Tibiaplateauteil wirkt. Das "Schalten" des Kniegelenkes geschieht also in dem angegebenen Bereich zwischen 20° und 40°. Das tatsächliche Ende des Kraft- und Formenschlusses beim zunehmenden Beugen des Gelenks hängt entscheidend von der Höhe des dorsal gelegenen Steges des Tibiaplateauteils ab und kann patientenindividuell festgelegt werden.

Es ist gemäß einer vorteilhaften Weiterbildung auch möglich, daß der ventral gelegene Steg stetig übergeht in den dorsal gelegenen Steg. Dies bedeutet herstellungstechnisch eine Vereinfachung.

Gemäß einer vorteilhaften Ausführungsform ist der Rotationsmechanismus zwischen dem Tibiaplateauteil und dem Tibiateil (Stielteil)aus einem am Tibiateil angeformten femurwärts weisenden Zapfen und einer entsprechend dimensionierten Bohrung in der Unterseite des Tibiaplateauteils, in welcher der Zapfen greift, ausgebildet. Diese Art der Verschwenkbarkeit eines Tibiaplateauteils auf dem Tibiateil ist per se bekannt.

Die Erfindung wird anhand eines Ausführungsbeispiels gemäß den Zeichnungsfiguren näher erläutert. Hierbei zeigt:
- Fig. 1: eine Seitenansicht eines Femurteils,
- Fig. 2: die Seitenansicht eines Tibiaplateauteils, und
- Fig. 3: Femur- und Tibiaplateauteil gemäß den Fig. 1 und 2 im Zusammenspiel.

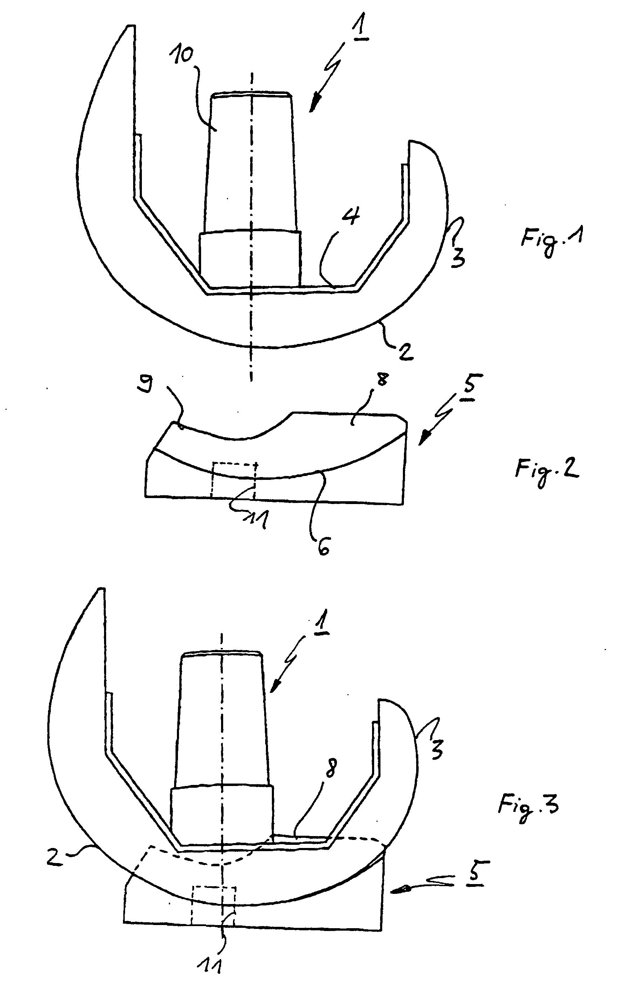

Das Femurteil 1 ist in Fig. 1 schematisch dargestellt. Es weist die beiden Gleitkufen auf, die ventral, also in Fig. 1 links, über eine Brücke (nicht dargestellt) miteinander verbunden sind. Zwischen sich begrenzen die Gleitkufen 2 und 3 einen von ventral nach dorsal verlaufenden Freiraum 4. Als Ankoppelungselement an ein in dem Femurknochen zu verankernden Stielteil ist vorliegend ein Steckkonus 10 vorgesehen.

Die Gleitkufen 2 und 3 des Femurteils 1 gemäß Fig. 1 gleiten und rollen ab auf Gleitbahnen 6, die im Tibiaplateauteil 5 ausgebildet sind. Das Tibiaplateauteil 5 wirkt zusammen mit einem im Tibiaknochen zu verankernden Stielteil (nicht dargestellt). Zwischen dem Tibiaplateauteil 5 und dem entsprechenden Stielteil ist ein Rotationsmechanismus vorgesehen, der per se bekannt ist und im wesentlich aus einem am Stielteil femurwärts weisend angeformten Zapfen besteht, der in eine Bohrung 11, in der Unterseite des Tibiaplateauteils 5 greift.

Beide Gleitbahnen 6 des Tibiaplateauteils 5 sind voneinander getrennt durch einen ventral gelegenen Steg 9 und einen dorsal gelegenen Steg 8. Vorliegend geht der ventral gelegene Steg 9 stetig über in den dorsal gelegenen Steg 8. Letzterer hat eine deutlich größere Höhe als der ventral gelegene Steg 9. Dies dient dem Zweck, daß der dorsal gelegene Steg 8 als mechanisches Schaltelement für das Kniegelenk dient. Wie sich nämlich aus Fig. 3 ergibt, in der das Femurteil 1 in gestreckter Lage des Kniegelenks auf dem Tibiaplateauteil 5 aufsitzend dargestellt ist, greift der dorsal gelegene, höhere Steg 8 in den Freiraum 4 zwischen den Gleitbahnen 2 und 3 des Femurteils 1. Hierdurch wird ein Kraft- und Formenschluß zwischen beiden Teilen hergestellt und eine Rotationsmöglichkeit des Femurteils 1 gegenüber dem Stielteil im Femur ist dadurch gegeben.

Mit zunehmender Beugung wird die Anlagefläche des Steges 8 an den Seitenkanten der Gleitkufen 2 und 3 kleiner, bis der Steg 8 außer Eingriff mit dem Freiraum 4 kommt. Dies ist exakt der Schaltpunkt zwischen dem technisch bedingten Bewegungsablauf hin zum anatomischen Bewegungsablauf. Bei Überschreiten eines Beugungswinkels im Bereich von 20° bis 40°, der patientenindividuell durch Auswahl eines entsprechenden Tibiaplateauteils 5 festgelegt werden kann, übernimmt nur der ventral gelegene, niedrigere Steg 9 die Führungsfunktion für die Gleitbahnen 2 und 3. Eine Rotationsmöglichkeit für das Femurteil 1 gegenüber dem Tibiaplateauteil 5 wird dadurch geschaffen, und zwar vollkommen unabhängig von dem Rotationsmechanismus zwischen dem Tibiaplateauteil 5 und dem Stielteil im Tibiaknochen.

## Patentansprüche

1. Kniegelenkendoprothese, bestehend aus
- einem mit einem in einem Femurknochen zu verankemden ersten Stielteil verbindbaren Femurteil (1), das mit zwei Gleitkufen (2, 3) versehen ist, die ventral über eine Brücke miteinander verbunden sind,
- einem mit einem in einem Tibiaknochen zu verankernden zweiten Stielteil zusammenwirkenden Tibiaplateauteil (5), in welchem zwei Gleitbahnen (6) ausgebildet sind, auf denen die Gleitkufen (2, 3) des Femurteils (1) eine Abroll- und Gleitbewegung ausführen können, wobei das Tibiaplateauteil (5) mittels eines Rotationsmechanismus auf dem zweiten Stielteil verschwenkbar gelagert ist,
**dadurch gekennzeichnet,**
**dass** bis zu einem vorgegebenen Beugungswinkel des Kniegelenks das Femurteil (1) und das Tibiaplateauteil (5) sich in einem Kraft- und Formenschluss befinden, derart, daß eine Rotationsbewegung des Femurteils (1) auf dem zweiten Stielteil aufgrund der verschwenkbaren Lagerung des Tibiaplateauteils (5) auf dem zweiten Stielteil ausführbar ist, und
**dass** dem Femurteil (1) bei zunehmender Beugung des Kniegelenks ab dem vorgegebenen Beugungswinkel eine zunehmende Rotation gegenüber dem Tibiaplateauteil (5) ermöglicht wird ohne Einschaltung des Rotationsmechanismus zwischen Tibiaplateauteil (5) und zweiten Stielteil.

2. Kniegelenkendoprothese nach Anspruch 1, bei der die Gleitkufen (2, 3) des Femurteils (1) zwischen sich einen von ventral nach dorsal verlaufenden Freiraum (4) begrenzen, und bei der beide Gleitbahnen (6) des Tibiaplateauteils (5) durch zwei von dorsal nach ventral ausgerichtet verlaufende Stege (8, 9) getrennt sind, wobei der dorsal gelegene Steg (8) eine solche Höhe aufweist, daß er bis zu dem vorgegebenen Beugungswinkel des Kniegelenks in den Freiraum (4) zwischen den Gleitkufen (2, 3) des Femurteils (1) greift und so den Kraft- und Formenschluß herstellt, und wobei der ventral gelegene Steg (9) lediglich eine solche Höhe hat, daß bei zunehmender Beugung des Kniegelenks eine zunehmende Rotation des Femurteils (1) gegenüber dem Tibiaplateauteil (5) ermöglicht wird.

3. Kniegelenkendoprothese nach Anspruch 1 oder 2, bei der der für ein Tibiaplateauteil (5) vorgegebene Beugungswinkel im Bereich zwischen 20° und 40° liegt, bis zu dessen Überschreiten der Formen- und Kraftschluß zwischen Femurteil (1) und Tibiaplateauteil (5) hergestellt wird.

4. Kniegelenkendoprothese nach einem der Ansprüche 1 bis 3, bei der der ventral gelegene Steg (9) stetig übergeht in den dorsal gelegenen Steg (8).

5. Kniegelenkendoprothese nach einem der Ansprüche 1 bis 4, bei der der Rotationsmechanismus zwischen dem Tibiaplateauteil (5) und dem Stielteil einen am Stielteil angeformten, femurwärts weisenden Zapfen und eine entsprechend dimensionierte Bohrung in der Unterseite des Tibiaplateauteils (5), in welche der Zapfen greift, aufweist.

## Claims

1. Knee joint endoprosthesis consisting of
- a femoral part (1) which can be connected to a first stem part to be anchored in a femoral bone and which is provided with two runners (2, 3), which are connected to one another ventrally via a bridge,
- a tibia plateau part (5) cooperating with a second stem part to be anchored in a tibia bone and in which two slide tracks (6) are formed, on which the runners (2, 3) of the femoral part (1) may execute a rolling-off and sliding movement, wherein the tibia plateau part (5) is pivotably mounted on the second stem part by means of a rotation mechanism,
**characterised in that** up to a preset bending angle of the knee joint, the femoral part (1) and the tibia plateau part (5) are situated in a frictional and positive connection, such that a rotational movement of the femoral part (1) can be executed on the second stem part due to the pivotable mounting of the tibia plateau part (5) on the second stem part, and **in that** for increasing bending of the knee joint from the preset bending angle, increasing rotation of the femoral part (1) with respect to the tibia plateau part (5) is facilitated without activating the rotation mechanism between tibia plateau part (5) and second stem part.

2. Knee joint endoprosthesis according to claim 1, in which the runners (2, 3) of the femoral part (1) between them define a space (4) running ventrally to dorsally, and in which both slide tracks (6) of the tibia plateau part (5) are separated by two bars (8, 9) running aligned dorsally to ventrally, wherein the dorsally placed bar (8) has such a height that it engages into the space (4) between runners (2, 3) of the femoral part (1) up to the preset bending angle of the knee joint and thus produces the frictional and positive connection, and wherein the ventrally placed bar (9) only has such a height that for increasing bending of the knee joint, increasing rotation of the femoral part (1) with respect to the tibia plateau part (5) is facilitated.

3. Knee joint endoprosthesis according to claim 1 or 2, in which the preset bending angle for a tibia plateau part (5) lies in the range between 20° and 40°, until which is exceeded the positive and frictional connection between femoral part (1) and tibia plateau part (5) is produced.

4. Knee joint endoprosthesis according to one of claims 1 to 3, in which the ventrally placed bar (9) changes continuously into the dorsally placed bar (8).

5. Knee joint endoprosthesis according to one of claims 1 to 4, in which the rotation mechanism between the tibia plateau part (5) and the stem part has a pin pointing in the direction of the femur moulded on the stem part and a correspondingly dimensioned bore in the lower side of the tibia plateau part (5), into which the pin engages.

## Revendications

1. Endoprothèse de genou comprenant
- un élément de fémur (1), qui est destiné à être assemblé avec une première partie de tige à ancrer dans un fémur naturel, et qui est muni de deux patins de glissement (2, 3), qui sont assemblés l'un à l'autre du côté ventral par l'intermédiaire d'un pont,
- un élément de plateau tibial (5), qui coopère avec une deuxième partie de tige à ancrer dans un tibia naturel et dans lequel sont réalisées deux voies de glissement (6) sur lesquelles les patins de glissement (2, 3) de l'élément de fémur (1) peuvent effectuer un mouvement de roulement et de glissement, l'élément de plateau tibial (5) étant logé de manière pivotante sur la deuxième partie de tige au moyen d'un mécanisme de rotation,
**caractérisée**
**en ce que**, jusqu'à un angle de flexion prédéfini de l'articulation du genou, l'élément de fémur (1) et l'élément de plateau tibial (5) sont accouplés par friction et emboîtement, de telle sorte qu'un mouvement de rotation de l'élément de fémur (1) peut être exécuté sur la deuxième partie de tige en raison du montage pivotant de l'élément de plateau tibial (5) sur la deuxième partie de tige, et
**en ce que** l'élément de fémur (1), en cas d'augmentation de la flexion de l'articulation du genou à partir de l'angle de flexion prédéfini, est autorisé à effectuer une rotation croissante par rapport à l'élément de plateau tibial (5) sans intervention du mécanisme de rotation entre l'élément de plateau tibial (5) et la deuxième partie de tige.

2. Endoprothèse de genou selon la revendication 1, dans laquelle les patins de glissement (2, 3) de l'élément de fémur (1) délimitent entre eux un espace libre (4) qui s'étend de ventral à dorsal, et dans laquelle les deux voies de glissement (6) dans l'élément de plateau tibial (5) sont séparées par deux traverses (8, 9), qui s'étendent en étant alignées dans le sens dorsal-ventral, sachant que la traverse (8) disposée du côté dorsal possède une telle hauteur qu'elle s'engage dans l'espace libre (4) entre les patins de glissement (2, 3) dans l'élément de fémur (1) jusqu'à l'angle de flexion prédéfini de l'articulation du genou et constitue ainsi le couplage par friction et emboîtement, et dans laquelle la traverse (9) située du côté ventral a uniquement une telle hauteur que, en cas d'augmentation de la flexion de l'articulation du genou, une rotation croissante de l'élément de fémur (1) par rapport à l'élément de plateau tibial (5) est rendue possible.

3. Endoprothèse de genou selon la revendication 1 ou 2, dans laquelle l'angle de flexion prédéfini pour un élément de plateau tibial (5) se situe dans la plage entre 20° et 40°, le couplage par friction et emboîtement étant maintenu entre l'élément de fémur (1) et l'élément de plateau tibial (5) jusqu'à ce que l'angle de flexion dépasse cette valeur.

4. Endoprothèse de genou selon l'une quelconque des revendications 1 à 3, dans laquelle la traverse (9) située du côté ventral se prolonge en continu vers la traverse (8) du côté dorsal.

5. Endoprothèse de genou selon l'une quelconque des revendications 1 à 4, dans laquelle le mécanisme de rotation entre l'élément de plateau tibial (5) et la partie de tige comporte un tenon, formé contre la partie de tige et orienté vers le fémur, et une forure, dimensionnée de manière correspondante dans la face inférieure de l'élément de plateau tibial (5) et destinée à recevoir le tenon.
